# EUROPEAN PATENT SPECIFICATION

(11) **EP 0 790 043 B1**
(45) Date of publication and mention of the grant of the patent: **19.11.2003**
(21) Application number: 96113579.5
(22) Date of filing: 23.08.1996
(51) Int. Cl.: A61F 2/24

(54) **Heart valve prosthesis**
Herzklappenprothese
Prothèse de valve cardiaque

(30) Priority: 14.02.1996 RU 96102902
(43) Date of publication of application: 20.08.1997
(73) Proprietor: ZAO MedEng, 440004 Penza (RU); Melnikov, Aleksandr Petrovich, Penza 440004 (RU)
(72) Inventor: Evdokimov, Sergey Vasilievich, 440056 Penza (RU); Melnikov, Aleksandr Petrovich, 440004 Penza (RU)
(74) Representative: Hano, Christian, Dipl.-Ing.

(56) References cited:
- EP-A- 0 327 790
- US-A- 4 863 459
- US-A- 5 197 980

## Description

The invention relates to a heart valve prosthesis comprising the features of the preamble of claim 1. Such a heart valve is known from EP-A-327 790.

The problem of creating heart valve prostheses capable of providing satisfactory substitutes for natural heart valves of humans damaged by congenital or acquired pathology have a history of more than 30 years.

During this long period many designs of heart valve prosthesis have been developed and tried, which function like a check valve ensuring direct blood flow when the closing element is in the opened position and preventing blood backflow when the closing element is in the closed position.

Due to their excellent hemodynamic parameters and the provision of central practically laminar flow the most commonly accepted valves in cardiac surgery today are the valves, which have a closing element comprising two semi-circular leaflets hinged to the body of the prosthesis while the hinge elements are either a projection on the body and a slot (recess) in the leaflet, e.g. US-A-4,863,459, or a recess in the body and a projection on the leaflet, e.g. US-A-4,276,658 or US-A-4, 689, 046.

The main differences in the constructions of such heart valve prostheses are connected with efforts to overcome the problem of thrombus formation at the prosthesis. In order to decrease the risk of thrombus formation one has to eliminate blood stagnation zones, ensure laminar blood flow and provide good washing of all elements of the valve.

An attempt to prevent thrombus formation at the implanted heart valve prosthesis has resulted in a heart valve prosthesis according to US-A-4,308,624 having an annular body inside which there is a closing element connected to the body through a means for turning said closing element from a closed position to an open position, and vice versa. The closing element comprises two arcuate leaflets. The downstream surface of each leaflet facing the backflow of blood is curved concave when the closing element is in a closed position.

The inner surface of the valve body is cylinder-shaped, its portion facing the direct blood flow having a smaller diameter than its other portion facing the backflow of blood. The meeting zone of these two portions serves as the valve seat. Two diametrically opposite flat portions run through the entire height of the valve body.

Each leaflet is connected to the valve body by a hinge mechanism. The hinge mechanism comprises two cooperating elements, one of which is located on the flat portions of the inner surface of the valve body while the other is located on the opposite sides of the closing element.

The element of the hinge mechanism located on the flat portions of the inner surface of the body is shaped as two oblong recesses which axes make up an angle of 20 degrees with the diametral plane of the body. The element of hinge mechanism located on the opposite sides of the closing element is shaped as spherical projections which engage the recesses located in the inner surface of the valve body and cooperate by their support surfaces with the support surfaces of the recesses.

When the valve opens the leaflets pivot in the hinges moving from a closed position to an open position. Due to the downstream concave surfaces of the leaflets a channel is created between the leaflets for passage of the direct blood flow. Hydrodynamic studies have shown that the structure of the direct blood flow between the leaflets is not homogeneous. Though in the plane running through the central axis of the body parallel to the flat portions of the body the blood flow is practically laminar and fills the whole section of the valve, yet in the plane perpendicular to the flat portions of the body a laminar flow exists only in the central part of the heart valve and there is a large blood stagnation zone in the hinge regions.

When the valve closes the leaflets pivot in the hinges moving from an open position to a closed position. During this action they interact with the valve seat located on the inner surface of the valve.

The absence of washing by blood flow in regions adjacent to hinges leads to higher risks of thrombus formation exactly at these places. The movement of leaflets along the blood flow during their opening and closing in oblong recesses does not solve the problem of thrombus formation because it does not eliminate stagnation zones and does not provide adequate washing of hinge mechanism. In addition the hinge in the form of a recess and projection that engages this recess does not reduce their thromboresistance but subjects the elements of blood to further damage when they are caught between the interacting friction surfaces of the recess and the projection.

An attempt to eliminate the above mentioned inadequacies was made with the heart valve prostheses which embody one common idea i.e. the realization of rotation of leaflets around the central axis of the valve body. In particular, in the heart valve prosthesis of US-A-4,274,437 leaflets of the prosthesis are connected to the valve body by means of hinges, the hinge elements of which are projections on the opposite sides of the leaflets that engage the recess in the inner surface of the valve body running along the entire periphery. In the heart valve prostheses of US-A-5,197,980 or EP 0 403 649 B1 the hinge elements are slots (recesses) located on the opposite sides of the leaflets that engage the annular projection on the inner surface of the valve body.

EP-A-0 327 790 discloses a prosthetic heart valve comprising an annular body along the entire periphery of which a projection is formed. Leaflets are mounted within the annular body so as to be pivotable between a closed position and an open position. In each leaflet recesses are formed near its vertices, the recesses cooperating with the annular projection. The dimensions of the recesses are such as to achieve a hinged engagement with the projection, with sufficient clearance to enable the leaflets to pivot freely between their closed positions and their open positions. The sole purpose of the clearance is to allow free pivoting of the leaflets.

During the valve closing the leaflets pivoting around the body projection interact by their downstream surface with the support surface of the projection and seal the valve.

During the movement of the leaflets from the open position into the closed one and vice versa, the leaflets have the possibility to rotate around the central axis of the valve body. This shall provide the movement of the stagnation zone along the periphery of the body and the elimination of constantly localized zones susceptible of thrombus formation.

Provision of the hinges in the form of a projection on the body and recesses in the leaflets reduces the friction surfaces and improves the washing of the elements of the hinge by blood flow.

However, there are no design elements that ensure compulsory rotation of the leaflets around the central axis of the valve body. Thus, it is very likely that such rotation will be contingent or will not happen at all. So the stagnation zones at the hinges will be localized and they will not be washed neither by the direct blood flow, nor by the blood backflow. Moreover, the projection at the inner surface of the valve body reduces the cross-section area of the valve orifice and contributes to the disturbance of the blood flow causing an increase of hydrodynamic resistance of the valve and an activation of the processes of thrombus formation.

It is the problem underlying the invention to create a heart valve prosthesis comprising a hinge mechanism that holds the leaflets within the valve body in such a way that for preventing thrombus formation localized stagnation zones inaccessible to blood washing are eliminated, while further improving the hemodynamic characteristics of the heart valve prosthesis and prolonging its lifetime.

This problem is solved by a heart valve prothesis comprising the features of claim 1.

Provision of the new hinge connection in the heart valve prosthesis in the form of projections at the leaflets and locating of the support means between them provides a limited backflow through the clearances between the portions of the outer side surface of the leaflets and the inner surface of the annular body. This limited backflow washes out adherent blood elements at the place of the leaflet hinge connection with the body and prevents thrombus formation at the heart valve elements. In addition, the jets of the backflow originating between said portions of the side surface of the leaflets and the inner surface of the annular body and directed in a certain way rotate a part of the blood located below the upstream end surface of the annular body thus eliminating blood stagnation zones and providing a washing effect before the prosthesis.

In a preferred embodiment the downstream surface of said support means is situated at the same level or upstream of said upstream end surface of said annular body.

In such a construction arrangement of the heart valve prosthesis, said support means used for the connection of the leaflets is located outside of the hydraulic orifice of the body. Thus there is the possibility to make it in such a way as to minimize reduction of the effective area of the hydraulic orifice of the body and effects of said element on the blood flow.

It is recommended to provide said support means along the entire periphery of the annular body. In such a case the leaflets obtain an additional degree of freedom, that is the possibility to rotate around the central axis of the annular body. As a result, the rotating blood part action due to the influence of said jets of blood backflow, located from the side of the upstream end surface of the annular body provides stable compulsory rotation of the leaflets around the central axis of the annular body in a required direction.

The stagnation zone adjacent to the hinges shall be washed by the blood backflow through the clearances between the side surface of the leaflet and the inner surface of the body as well as by the direct blood flow when the leaflet rotates around the central axis of the body.

The location of said support means on the upstream end surface of the annular body reduces leaflet exposure relative to the downstream end surface of the body when the leaflets are in the open position, thus reducing the risk of their contact with surrounding heart structures which could lead to a jamming of the leaflets in their open position.

In case when heart valve prosthesis is used for patients with a small ventricle or there are some other ventricular obstacles that might impede a leaflet rotation around the central axis of the annular valve body, it is better to make said support means in the form of two protrusions located at diametrically opposite portions of the upstream end surface of the body so that the side surfaces of said support means face each other and their projections on the plane perpendicular to the central axis of the annular body are in fact the arcs of the circumference with a centre located on the central axis of the annular body and the upstream support surfaces of each support means must have stops at their ends for being able to interact with respective leaflet projections. The length of the upstream support surface of said support means, limited by said stops, should be from 0.05 to 0.25 of the length of the inner surface of the annular body.

In such a construction arrangement of a heart valve prosthesis the leaflets are also connected to the valve body by the hinge mechanism, elements of which are the projections on the leaflets locating between them said support means in the form of two stepped protrusions on the upstream end surface of the valve body. They also have an additional degree of freedom and can rotate around the central axis of the annular body, not a full turn but only in an oscillating movement. With such movement, the hinge mechanism zone also moves but the amplitude of this movement is limited by the stops on the upstream surface of said support means. Surgeon gains the possibility to orientate the prosthesis in the ventricle in such a way as to avoid an open leaflet contact with surrounding heart structures.

In order to provide the compulsory oscillating movement of leaflets around the central axis of the body, the downstream support surfaces of each protrusion located on diametrically opposite portions of the upstream end surface of the body should be made in such a way so that their logitudinal axes are at an angle relative to the central axis of the body and for the surfaces located on the opposite protrusion the angle modulus is equal but with the opposite sign. In such a construction arrangement of a heart valve prosthesis the downstream support surfaces of the protrusion located on the upstream end surface of the body form a slanting surface relative to the movement direction of the blood backflow and due to blood pressure on closing leaflets, they somewhat skid along the slant downstream support surface in one direction and due to the forces of the rotating part of the direct blood flow the opening leaflets turn in the opposite direction. The turn of the leaflets as a result of this oscillating movement is limited by the stops located on the ends of the upstream support surfaces of said annular support means.

It is recommended to make at least part of the inner surface of the annular body located from the side of direct blood flow in the form of a confuser surface with which the projections interact which are located on portions of the side surface of the leaflets having a clearance relative to the side surface of said support means and with the arc radius of the side surface of said support means being equal or greater than the radius of the inner surface of the annular body.

In such a construction arrangement of the prosthesis, said support means does not protrude inside the body, so there is no reduction of the effective area of the orifice, hence there are lower energy losses during the passage of direct blood flow and better hemodynamic characteristics of the heart valve prosthesis.

It is expedient that each leaflet has two portions of outer side surfaces made with a clearance relative to the side surface of said support means located on the opposite sides of the outer side surface, each of them having at least two said projections. One of these projections interacts by its support portion on the upstream surface with downstream support surface of said support means, while the other interacts by its support portion on the downstream surface with the upstream support surface of said support means. The support surfaces located on said leaflet projections and interacting with the support surfaces of said support means are made convex.

Provision of the portions on the opposite sides of the outer side surface with a clearance relative to said support means enables washout of adherent blood elements from each zone where a leaflet is hinged to the body. Provision of projections on these portions enables not only retaining and pivoting of the leaflet within the body but also fixing of the clearance between the side surface of the leaflet and the inner surface of the body which determines the rate of blood backflow that washes the hinge adjacent zone.

Provision of convex support surfaces of said leaflet projections which interact with the support surfaces of said support means, enables rolling friction interaction between them which ensures minimal wear out of the interacting surfaces and increases the reliability and lifetime of the heart valve. In addition this prevents a trauma of the formed blood elements.

In a further preferred embodiment at least on one of said leaflets a channel is provided, the surface of the channel crossing the contact surface of said leaflet to allow a restricted blood backflow when the leaflets are in the closed position.

In such a construction arrangement of the prosthesis, the limited blood backflow, passing through said channel, washes those zones of the valve surfaces where a higher probability of the adhesion of the formed blood elements exists.

It is expedient that each leaflet has at least one said channel in order to allow a limited blood backflow. Said channel is located at the contact surface and is bounded on one side by the inner surface of the annular body. The total area of said channels for the passage of blood backflow and the clearances between the outer side surface and the inner surface of the body and the side surface of said support means should be from 0,2 to 8% of the orifice area of the body.

In such a construction arrangement of the prosthesis the backflow washes not only the downstream surface of the leaflets but also the interacting surfaces of said support means and of the projections on the side surface of the leaflets. In addition the stream of the blood backflow reaches the surfaces of the body and washes the threads of fibrin away which could spread from the sewing ring to the side surface of the leaflets.

The optimum rate of the limited backflow is reached when this flow is strong enough, but the volume of blood passing back should not exceed 20 % of the volume of blood passing through in direct direction, otherwise the energy losses at the valve are considered to be unacceptably high. In order to obtain the optimum rate of blood backflow it is necessary that the aggregate area of said channels should be within 0.2 to 8 % of the area of the body orifice.

The heart valve prosthesis, made according to the invention, allows to reduce the probability of thrombus formation, has improved hemodynamic characteristics and higher reliability of valve construction. It finds most utility when applied for replacement of degenerative natural aortic and mitral heart valves in humans, and will meet equal success when used for replacing a damaged tricuspid valve.

In the following embodiments of the invention are described by means of the accompanying drawings, wherein
- Fig.1: is an enlarged view of a first embodiment of a heart valve prosthesis with its leaflets in a closed position as viewed from the downstream side with respect to the direct blood flow;
- Fig.2: is a partly sectional view taken along lines II-II of Fig.1;
- Fig.3: is an enlarged view of a second embodiment of the heart valve prosthesis with its leaflets in an open position as viewed from the upstream side with respect to the direct blood flow;
- Fig.4: is a partly sectional view taken along lines IV-IV of Fig.3;
- Fig.5: is an enlarged fragmentary sectional view of the heart valve prosthesis taken along lines V-V of Fig.4,;
- Fig.6: is an enlarged view of a third embodiment of the heart valve prosthesis with its leaflets in a closed position as viewed from the downstream side with respect to the direct blood flow;
- Fig.7: is a partly sectional view taken along lines VII-VII of Fig.6;

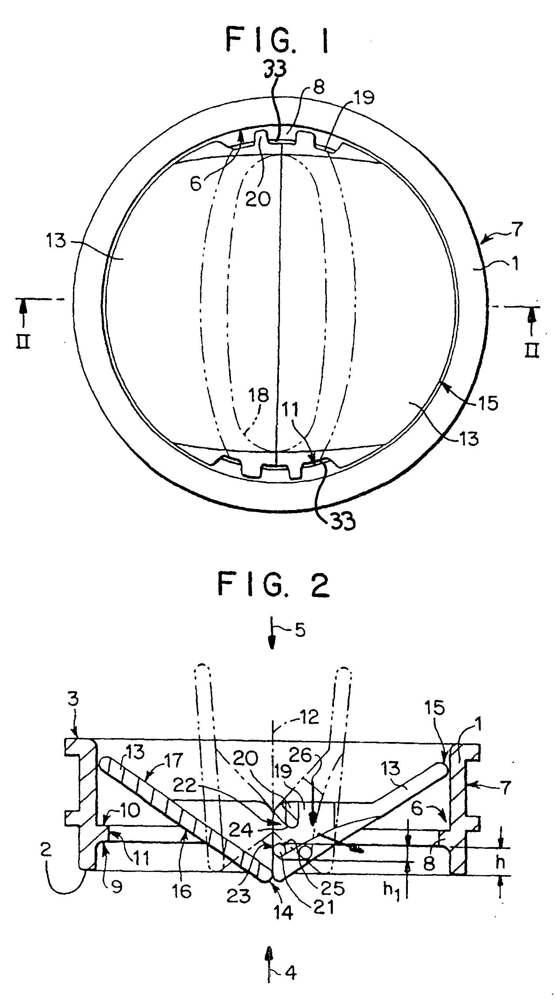

In Fig.2, Fig.4 and Fig.7 broken lines are used to show the leaflets in the open position.

The heart valve prostheses shown in Fig. 1 to 7 are used for replacement of diseased natural aortic or mitral heart valves.

The first embodiment of the heart valve prosthesis shown in Fig.1 and Fig.2 comprises an annular body 1, having an upstream end surface 2 and a downstream end surface 3, facing the direct blood flow 4 and the blood backflow 5, respectively, an inner surface 6 and an outer surface 7. In the interior of the annular body 1 an annular flange 8 is provided having an upstream support surface 9, a downstream support surface 10, and a side surface 11, facing the central axis 12 of the annular body 1. The annular flange 8 runs along the entire periphery of the innner surface of the annular body 1.

Within the annular body 1 two leaflets 13 are mounted pivotable between a closed position in which the blood backflow 5 is restricted and an open position which enables the passage of the direct blood flow 4. Each leaflet 13 comprises a contact surface 14 cooperating with the contact surface of the other leaflet in the closed position, an outer side surface 15, an upstream surface 16 and a downstream surface 17. A portion 18 of the downstream surface 17 of each leaflet 13 is formed concave to allow the passage of the direct blood flow 4 between the leaflets 13 when they are in the open position.

The outer side surface 15 of each leaflet 13 comprises two portions 19 arranged with clearance 33 relative to the side surface 11 of the flange 8 of the annular body 1 and located on the opposite sides of the outer side surface 15 of each leaflet 13.

Each portion 19 comprises an upstream projection 20 and a downstream projection 21 the space therebetween being limited by an upstream support surface 22 and and a downstream support surface 23 forming support portions 24 and 25, respectively, which interact with the downstream surface 10 and the upstream surface 9 of the flange 8 of the annular body, respectively. The support portions 24 and 25 are smoothly convex. The distance "h" of the upstream support surface 9 of the flange 8 from the upstream end surface 2 is greater than the axial dimension "h₁" of the projections 21, forming a protective barrier for said projections 21 of the leaflets 13, which prevents the spread of pseudointima covering the sewing ring into the hinge mechanism that holds leaflets 13 within the valve body 1.

The first embodiment of the heart valve prosthesis shown in Fig. 1 and 2 functions as follows. In the closed position the leaflets 13, which are interacting with the downstream surface 10 of the flange 8 of the annular body 1 by the support portions 24 of the projections 20 and the contact surfaces 14, close the orifice of the annular body 1 so as to restrict the blood backflow 5 through the prosthesis. At this moment due to excessive pressure jets 26 of the restricted blood backflow 5 are created which flow through the clearances 33 between the portions 19 of the outer side surface 15 of the leaflets 13 and the inner surface 6 of the annular body 1. This provides the washing out of adherent blood elements in the zone where the leaflets 13 are hinged to the annular body 1 and thus preventing thrombus formation at the elements of the heart valve prosthesis. Furthermore, due to the definite shape and dimensions of the projections 20, the jets 26 of the blood backflow 5 at one of the portions 19 of said outer side surface 15 of the leaflets 13 reflect from the downstream support surface 10 of the flange 8 of the annular body 1 and move at an angle relative to the central axis 12 of the annular body 1. As a result a part of blood volume at the upstream end surface 2 of the annular body 1 begins to rotate in a certain direction thus eliminating stagnation zones and providing washing of the heart structures in front of the prosthesis.

When an excessive pressure is created from the side of the upstream end surface 2 of the annular body 1, the leaflets 13 interacting by the support portions 25 on the projection 21 with the upstream surface 9 of the flange 8, open to allow the passage of the direct blood flow 4.

At this moment the rotating blood volume generated by the jets 26 of the blood backflow 5 passing through the orifice of the annular body 1 turns the opening leaflets 13 to some angle around the central axis 12. This provides stable compulsory rotation of the leaflets 13 around the entire periphery of the inner surface 6 of the annular body 1.

At the same time the stagnation zone located at the region of hinges shall be washed by the blood backflow 5 through the clearances 33 between the outer sid surface 15 of the leaflets 13 and the inner surface 6 of the annular body 1 as well as by the direct blood flow 4 because of the rotation of the leaflets 13 around the central axis of the annular body 1.

The direct blood flow 4 between the leaflets 13 is secured by forming parts 18 of the downstream surfaces 17 of the leaflets 13 smoothly concave.

When an excessive pressure is created from the side of the downstream end surface 3 of the annular body 1, the leaflets 13 interacting by the support portions 24 of the projections 20 with the downstream surfaces 10 of the flange 8 of the annular body 1, pivot into the closed position and restrict the blood backflow 5.

Provision of the smoothly convex support surfaces 22, 23 on the support portions 24 and 25 of the leaflets 13 ensures a rolling friction interaction thereof which leads to minimum wear of the interacting surfaces and better reliability and longer lifetime of the valve and in addition prevents trauma of the formed elements of blood.

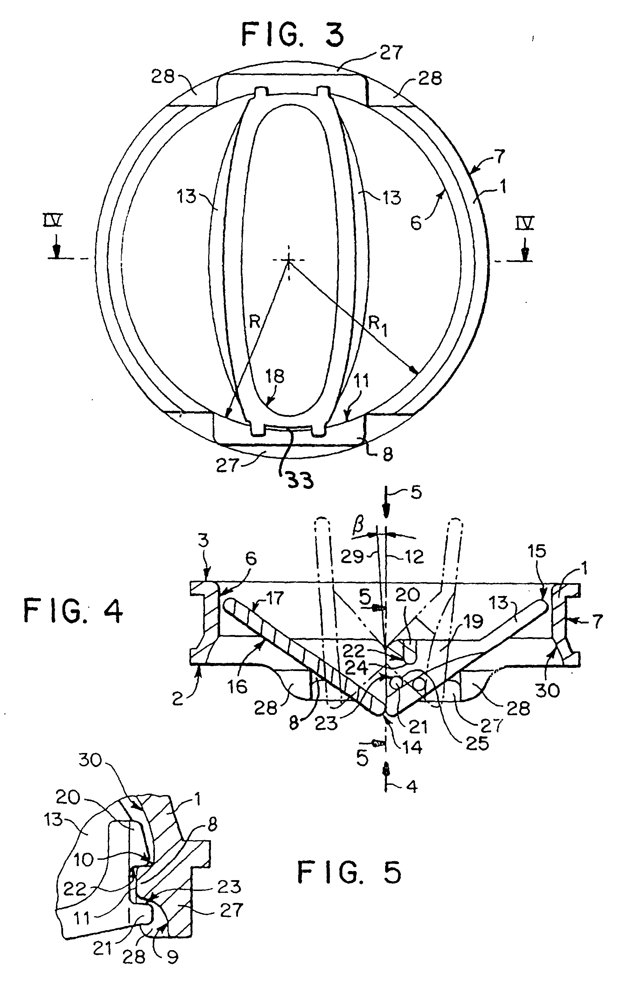

The second embodiment of the heart valve prosthesis shown in Fig.3 to Fig.5 comprises an annular body 1, having an upstream end surface 2 and a downstream end surface 3, facing the direct blood flow 4 and the blood backflow 5, respectively, an inner surface 6 and an outer surface 7.

The annular body 1 has two stepped protrusions 27 located on diameterically opposite portions of the upstream end surface 2 of the annular body 1, each of the protrusions having an upstream support surface 9 and a downstream support surface 10 facing the direct blood flow 4 and the blood backflow 5, respectively, and a side surface 11. The downstream support surfaces 10 of said stepped protrusions 27 lie higher upstream of the direct blood flow 4 than the upstream end surface 2, and the ends of the upstream support surfaces 9 thereof are provided with stops 28. The length of each upstream surface 10 between the stops 28 amounts between 0.05 to 0.25 of the length of the inner surface 6 of the annular body 1. In addition, the longitudinal axes 29 of the downstream support surfaces 10 of the stepped protrusions 27 are at an angle relative to the central axis 12 of the annular body 1. The values of the slant angles "β" of said longitudinal axes 29 of the downstream surfaces 10 of the stepped protrusions 27 located on diametrically opposite portions of the upstream end surface 2 of the annular body 1 are equal by modulus but with opposite sign. The side surfaces 11 of each stepped protrusion 27 face each other and their projections on the plane perpendicular to the central axis 12, are arcs of a circumference with its centre located on said central axis 12.

Within the annular body 1 two leaflets 13 are mounted so as to pivot between a closed position, restricting the blood backflow 5, and an open pasition enabling the passage of the direct blood flow 4. Each leaflet 13 has a contact surface 14 cooperating with the contact surface of the other leaflet 13 in the closed position, an outer side surface 15, an upstream surface 16 and a downstream surface 17 facing the direct blood flow 4 and the blood backflow 5, respectively. A part 18 of the downstream surface 17 of each leaflet is made concave to allow the passage of the direct blood flow 4 between the leaflets 13 in their open position. The outer side surface 15 of each leaflet 13 has two portions 19 formed with a clearance 33 relative to the side surface 11 of the protrusions 27 of the annular body 1 and located on the opposite sides of the outer side surface 15 of the leaflets 13.

Each portion 19 has two projections 20 and 21 having an upstream surface 22 and a downstream surface 23, respectively, with two support portions 24 and 25, respectively, which interact with the corresponding downstream support surface 10 or upstream support surface 9 of the protrusions 27 of the annular body 1. The support portions 24 and 25 of the projections 20 and 21 are made smoothly convex. A part of the inner surface 6 of the annular body 1 extending from the side of the direct flow 4 is formed like a confuser surface 30 with which interact the projections 20 located on the portions 19 of the side surface 15 of the leaflets 13. The radius "R" of the side surfaces 11 of the stepped protrusions 27 is equal to the radius "R₁" of the inner surface 6 of the annular body 1.

The protrusions do not protrude inside the valve body 1. Therefore there is no reduction of the effective area of the body orifice, hence there are lower energy losses during the passage of direct blood flow 4 and better hemodynamic characteristics of the heart valve prosthesis.

The second embodiment of the heart valve prosthesis shown in Fig.3 to 5 functions like the prosthesis shown in Fig.1 and 2 with the exception that due to the action of forces from the side of the rotating part of the direct blood flow 4, because of the effects of the back flow jets 26, the opening leaflets 13 turn around the central axis 12 of the annular body 1 until the projections 21 interact with the stops 28 of the upstream surfaces 9 of the stepped protrusions 27. During valve closing the leaflets 13 skid along the slanting downstream support surface 10 due to pressure and at the same time also turn around the central axis 12 until the interaction of the projections 21 with the stops 28. The direction of the slant of the downstream support surfaces 10 of the stepped protrusions 27 is chosen in such a way that the leaflets 13 turn around the central axis 12 in one direction during valve opening and in the opposite one during valve closing.

In such a construction arrangement of the heart valve prosthesis the leaflets 13 also have an additional degree of freedom and can rotate around the central axis 12 of the annular body 1, not a full turn but only in the form of an oscillating movement. With such movement, the hinge mechanism zone also moves but the amplitude of this movement is limited by the stops 28 on the upstream support surfaces 9 of the stepped protrusions 27. Therefore surgeon gains the possibility to orientate the prosthesis in ventricle in such a way as to avoid the contact of the leaflets 13 with surrounding heart structures during valve opening.

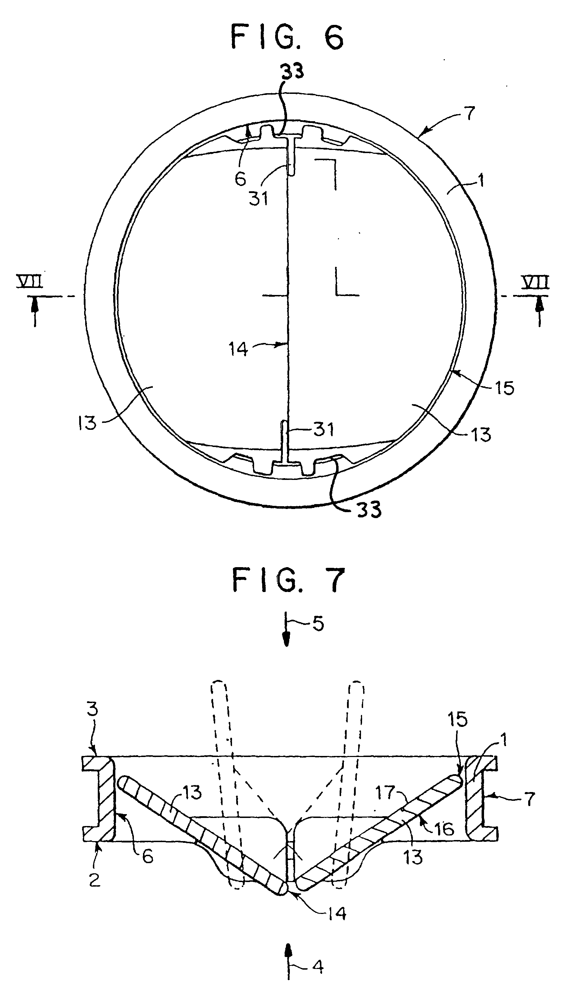

The third embodiment of the heart valve prosthesis shown in Fig.6 and 7 comprises an annular body 1, having an upstream end surface 2 and a downstream end surface 3, facing the direct blood flow 4 and the blood backflow 5, respectively, an inner surface 6 and an outer surface 7. Within the annular body 1 two leaflets 13 are mounted pivotable between a closed position in which the blood backflow 5 is restricted and an open position which enables the passage of the direct blood flow 4. Each leaflet 13 comprises a contact surface 14 cooperating with the contact surface of the other leaflet in the closed position, an outer side surface 15, an upstream surface 16 and a downstream 17 surface.

One channel 31 is provided on each leaflet 13, which crosses the contact surface 14 and is located on one side of the outer side surface 15. The channel 31 is limited on one side by the inner surface 6 of the annular body 1. The total area of said channels 31 and of the clearances 33 between the outer side surface 15 and the inner surface 6 of the annular body 1 is chosen in such a way that the limited blood backflow will not not exceed 20 % of the volume of the direct blood flow 4 and is from 0.2 to 8 % of the orifice area of the annular body 1.

In such a construction arrangement of the prosthesis, the limited blood backflow 5 passing through the channels 31 when the leaflets 13 are in the closed position, washes the zones of the downstream surfaces 17 of the leaflets 13 where exists high probability of adhesion of the formed elements of blood. In addition the jets of the blood backflow 5, passing through the channels 31, reach the upstream end surface 2 of the annular body 1 and wash away the threads of fibrin which could spread from the surrounding heart structures to the side surface of the leaflets 13.

The laboratory studies have shown the minimal pressure losses across the heart valve prostheses made up in accordance with the invention during the passage of the direct flow of fluid with the optimum volume of back flow.

The tests at the accelerated life stand for a period equal to 50 years of in vivo service of the prosthesis have proved high durability and reliability of the prostheses. There have been no incident of disruption of the prosthesis. Wear of the interacting elements of the prostheses had been insignificant and hemodynamic and functional characteristics of the heart valve prostheses remained practically the same.

After the overall laboratory studies samples of the heart valve prosthesis made up in accordance with the invention have been submitted for the extended clinical tests, which proved high thromboresistance, hemodynamic effectiveness and reliability of the heart valve prostheses that follow the invention design for the replacement of damaged natural heart valves in humans.

## Claims

1. A heart valve prosthesis comprising
- an annular body (1) having an upstream end surface (2) facing the direct blood flow (4), a downstream end surface (3) facing the blood backflow (5), an inner surface (6) and an outer surface (7),
- support means (8) having an upstream support surface (9), a downstream support surface (10) and a side surface (11) facing the central axis (12) of said annular body (1), and
- leaflets (13) pivotally mounted within said annular body (1) so as to pivot between a closed position in which the blood backflow (5) is restricted and an open position which allows the passage of the direct blood flow (4), each leaflet (13) having a contact surface (14) cooperating with the contact surface (14) of the other leaflet (13) in the closed position, an outer side surface (15), an upstream surface (16), a downstream surface (17) and support portions (24, 25) respectively interacting with said downstream support surface (10) and said upstream support surface (9) of said support means (8; 27) of said annular body (1), respectively,
- at least one portion (19) of the outer side surface (15) of at least one of said leaflets (13) being formed such that, when the leaflets (13) are in the closed position, a clearance (33) is provided between said at least one portion (19) and said side surface (11) of said support means (8) of said annular body (1) through which a restricted blood backflow (5) can flow,
**characterized in that**
- at said at least one portion (19) a downstream projection (20) projecting from said outer side surface (15) and an upstream projection (21) projecting from said outer side surface (15) are provided, the upstream surface (22) of said downstream projection (20) and the downstream surface (23) of said upstream projection (21) forming the support portions (24, 25),
- wherein in the closed position, the downstream projection (20) and the clearance (33) provide jets of restricted blood flow which reflect from the downstream support surface (10) and move at an angle relative to the central axis, so that the blood back flow rotates to eliminate blood stagnation zones in the prosthesis.

2. A heart valve prosthesis according to claim 1, charaterized in that said downstream surface (10) of said support means (27) is situated at the same level or upstream of said upstream end surface (2) of said annular body (1).

3. A heart valve prosthesis according to claim 1 or claim 2, **characterized in that** two of said portions (19) are located on opposite sides of the outer side surface (15) of the leaflets (13).

4. A heart valve prosthesis according to any one of claims 1 to 3, **characterized in that** the support portions (24, 25) are smoothly convex.

5. A heart valve prosthesis according to any one of claims 1 to 4, **characterized in that** at least a part of said inner surface (6) of said annular body (1) which is located downstream from the support means (8) of the annular body (1) has the form of a confusor surface (30) cooperating with the projections (20) which interact with the downstream support surfaces (10) of said support means (8).

6. A heart valve prosthesis according to any one of claims 1 to 5, **characterized in that** said support means (8) is arranged along the entire periphery of said annular body (1), at least a part (18) of the downstream surface (17) of each leaflet (13) being smoothly concave to allow the passage of the direct blood flow (4) between the leaflets (13) in their open position.

7. A heart valve prosthesis according to any one of claims 1 to 5, **characterized in that** said support means (8) is formed by two protrusions (27) located on diametrically opposite portions of said upstream end surface (2) of said annular body (1), the side surfaces (11) of the two protrusions (27) facing each other and the projections of said side surfaces (11) on a plane perpendicular to the central axis (12) of said annular body (1) being arcs of a circle the center of which is located on said central axis (12).

8. A heart valve prosthesis according to claim 7, **characterized in that** at the ends of the upstream support surface (10) of each protrusion (27) stops (28) are provided for cooperating with said projections (21) interacting with said upstream support surfaces (10), the length of each upstream surface (10) between said stops (28) ranging from 0.05 to 0.25 of the length of the inner surface (6) of said annular body (1).

9. A heart valve prosthesis according to claim 1, **characterized in that** in at least on one of said leaflets (13) a channel (31) is provided, the surface of the channel (31) crossing the contact surface (14) of said leaflet (13) to allow a restricted blood backflow (5) when the leaflets (13) are in the closed position.

10. A heart valve prosthesis according to claim 9, **characterized in that** at least one channel (31) is provided at the contact surface (14) of each leaflet (13), the channel (31) being bounded on one side by the inner surface (6) of said annular body (1) and **in that** the overall area of said at least one channel (31) and the clearances (33) between the outer side surface (15) of the leaflets (13) and the inner surface (6) of said annular body (1) ranges from 0.2 to 8% of the orifice area of said annular body (1).

## Patentansprüche

1. Herzklappenprothese
- mit einem Ringkörper (1), der eine stromaufseitige Stirnfläche (2), die dem direkten Blutstrom (4) zugewandt ist, eine stromabseitige Stirnfläche (3), die dem Blutrückstrom (5) zugewandt ist, eine Innenfläche (6) und eine Außenfläche (7) aufweist,
- mit einer Halteeinrichtung (8), die eine stromaufseitige Haltefläche (9), eine stromabseitige Haltefläche (10) und eine Seitenfläche (11) hat, die der zentralen Achse (12) des Ringkörpers (1) zugewandt ist, und
- mit Klappensegeln (13), die in dem Ringkörper (1) so angelenkt sind, dass sie zwischen einer Schließstellung, in der der Blutrückstrom (5) eingeengt ist, und einer Offenstellung, die den Durchgang des direkten Blutstroms (4) erlaubt, verschwenken, wobei jedes Klappensegel (13) eine Kontaktfläche (14), die mit der Kontaktfläche (14) des anderen Klappensegels (13) in der Schließstellung zusammenwirkt, eine äußere Seitenfläche (15), eine stromaufseitige Fläche (16), eine stromabseitige Fläche (17) und Halteabschnitte (24, 25) aufweist, die jeweils mit der stromaufseitigen Haltefläche (10) beziehungsweise der stromabseitigen Haltefläche (9) der Halteeinrichtung (8, 27) des Ringkörpers (1) zusammenwirken,
- wobei wenigstens ein Teil (19) der äußeren Seitenfläche (15) wenigstens eines Klappensegels (13) so geformt ist, dass, wenn die Klappensegel (13) sich in der Schließstellung befinden, zwischen dem wenigstens einen Teil (19) und der Seitenfläche (11) der Halteeinrichtung (8) des Ringkörpers (1) ein Freiraum (33) vorgesehen ist, durch den ein eingeschränkter Blutrückstrom (5) strömen kann,
**dadurch gekennzeichnet,**
- **dass** an dem wenigstens einen Teil (19) ein stromabseitiger Vorsprung (20), der von der äußeren Seitenfläche (15) vorsteht, und ein stromaufseitiger Vorsprung (21), der von der äußeren Seitenfläche (15) vorsteht, vorgesehen sind, wobei die stromaufseitige Fläche (22) des stromabseitigen Vorsprungs (20) und die stromabseitige Fläche (23) des stromaufseitigen Vorsprungs (21) die Halteabschnitte (24, 25) bilden, und
- wobei in der Schließstellung der stromabseitige Vorsprung (20) und der Freiraum (33) Strahlen des verengten Blutstroms bereitstellen, die von der stromabseitigen Haltefläche (10) reflektiert werden und sich in einem Winkel bezüglich der zentralen Achse so bewegen, dass der Blutrückstrom rotiert, um Blutstagnationszonen in der Prothese zu beseitigen.

2. Herzklappenprothese nach Anspruch 1, **dadurch gekennzeichnet, dass** die stromabseitige Fläche (10) der Halteeinrichtung (27) sich auf der gleichen Höhe wie die stromaufseitige Stirnfläche (2) des Ringkörpers (1) oder stromauf von ihr befindet.

3. Herzklappenprothese nach Anspruch 1 oder Anspruch 2, **dadurch gekennzeichnet, dass** sich zwei der Teile (19) auf gegenüberliegenden Seiten der äußeren Seitenfläche (15) der Klappensegel (13) befinden.

4. Herzklappenprothese nach einem der Ansprüche 1 bis 3, **dadurch gekennzeichnet, dass** die Halteabschnitte (24, 25) leicht konvex sind.

5. Herzklappenprothese nach einem der Ansprüche 1 bis 4, **dadurch gekennzeichnet, dass** wenigstens ein Teil der Innenfläche (6) des Ringkörpers (1), der sich stromab von der Halteeinrichtung (8) des Ringkörpers (1) befindet, die Form einer Konfusorfläche (30) hat, die mit den Vorsprüngen (20) zusammenarbeitet, die mit den stromabseitigen Halteflächen (10) der Halteeinrichtung (8) zusammenwirken.

6. Herzklappenprothese nach einem der Ansprüche 1 bis 5, **dadurch gekennzeichnet, dass** die Halteeinrichtung (8) längs des gesamten Umfangs des Ringkörpers (1) angeordnet ist, wobei wenigstens ein Teil (18) der stromabseitigen Fläche (17) jedes Klappensegels (13) leicht konkav ist, um den Durchgang des direkten Blutstroms (4) zwischen den Klappensegeln (13) in ihrer Offenstellung zu erlauben.

7. Herzklappenprothese nach einem der Ansprüche 1 bis 5 **dadurch gekennzeichnet, dass** die Halteeinrichtung (8) von zwei Vorsprüngen (27) gebildet wird, die sich an diametral gegenüberliegenden Abschnitten der stromaufseitigen Stirnfläche (2) des Ringkörpers (1) befinden, wobei die Seitenflächen (11) der beiden Vorsprünge (27) einander zugewandt sind und die Vorsprünge der Seitenflächen (11) auf einer Ebene senkrecht zur zentralen Achse (12) des Ringkörpers (1) Kreisbögen sind, deren Zentrum sich auf der zentralen Achse (12) befindet.

8. Herzklappenprothese nach Anspruch 7, **dadurch gekennzeichnet, dass** an den Enden der stromaufseitigen Haltefläche (10) eines jeden Vorsprungs (27) Anschläge (28) für ein Zusammenarbeiten mit den Vorsprüngen (21) vorgesehen sind, die mit den stromaufseitigen Halteflächen (10) zusammenwirken, wobei die Länge einer jeden stromaufseitigen Fläche (10) zwischen den Anschlägen (28) in einem Bereich von 0,05 bis 0,25 der Länge der Innenfläche (6) des Ringkörpers (1) liegt.

9. Herzklappenprothese nach Anspruch 1, **dadurch gekennzeichnet, dass** in wenigstens einem der Klappensegel (13) ein Kanal (31) vorgesehen ist, wobei die Oberfläche des Kanals (31) die Kontaktfläche (14) des Klappensegels (13) kreuzt, um einen eingeschränkten Blutrückstrom (5) zu ermöglichen, wenn sich die Klappensegel (13) in der Schließstellung befinden.

10. Herzklappenprothese nach Anspruch 9, **dadurch gekennzeichnet, dass** wenigstens ein Kanal (31) an der Kontaktfläche (14) eines jedes Klappensegels (13) vorgesehen ist, wobei der Kanal (31) auf einer Seite von der Innenfläche des Ringkörpers (1) begrenzt ist, und dass die Gesamtfläche des wenigstens einen Kanals (31) und der Freiräume (33) zwischen der äußeren Seitenfläche (15) der Klappensegel (13) und der Innenfläche (6) des Ringkörpers (1) in einem Bereich von 0,2 bis 8% der Öffnungsfläche des Ringkörpers (1) liegt.

## Revendications

1. Prothèse de valve cardiaque comprenant
- un corps annulaire (1) ayant une surface d'extrémité en amont (2) faisant face au flux sanguin direct (4), une surface d'extrémité en aval (3) faisant face au reflux sanguin (5), une surface interne (6) et une surface externe (7),
- des moyens supports (8) ayant une surface support en amont (9), une surface support en aval (10) et une surface latérale (11) faisant face à l'axe central (12) dudit corps annulaire (1), et
- des feuillets (13) montés pivotant à l'intérieur du corps annulaire (1), de façon à pivoter entre une position fermée dans laquelle le reflux sanguin (5) est limité et une position ouverte qui permet le passage du flux sanguin direct (4), chaque feuillet (13) possédant une surface de contact (14) qui coopère avec la surface de contact (14) de l'autre feuillet (13) dans la position fermée, une surface latérale externe (15), une surface en amont (16), une surface en aval (17) et des portions supports (24, 25) interagissant respectivement avec ladite surface support en aval (10) et ladite surface support en amont (9), respectivement desdits moyens supports (8; 27) dudit corps annulaire (1),
- au moins une portion (19) de la surface latérale externe (15) d'au moins l'un desdits feuillets (13) étant formée de sorte que lorsque les feuillets (13) sont dans la position fermée, un dégagement (33) est fourni entre ladite au moins une portion (19) et ladite surface latérale (11) desdits moyens supports (8) dudit corps annulaire (1) à travers lequel un reflux sanguin limité (5) peut s'écouler,
**caractérisée en ce que**
- au niveau de ladite au moins une portion (19), une projection en aval (20) se projetant de ladite surface latérale externe (15) et une projection en amont (21) se projetant de ladite surface latérale externe (15) sont prévues, la surface en amont (22) de ladite projection en aval (20) et la surface en aval (23) de ladite projection en amont (21) formant les portions supports (24, 25),
- dans laquelle dans la position fermée, la projection en aval (20) et le dégagement (33) fournissent des giclées de flux sanguin limité qui se réfléchissent à partir de la surface support en aval (10) et se déplacent selon un angle par rapport à l'axe central, de telle sorte que le reflux sanguin tourne afin d'éliminer des zones de stagnation sanguines dans la prothèse.

2. Prothèse de valve cardiaque selon la revendication 1, **caractérisée en ce que** ladite surface en aval (10) desdits moyens supports (27) est située au même niveau ou en amont de ladite surface d'extrémité en amont (2) dudit corps annulaire (1).

3. Prothèse de valve cardiaque selon la revendication 1 ou la revendication 2, **caractérisée en ce que** deux desdites portions (19) sont situées des côtés opposés de la surface latérale externe (15) des feuillets (13).

4. Prothèse de valve cardiaque selon l'une quelconque des revendications 1 à 3, **caractérisée en ce que** les portions supports (24, 25) sont régulièrement convexes et lisses.

5. Prothèse de valve cardiaque selon l'une quelconque des revendications 1 à 4, **caractérisée en ce que**, au moins une partie de ladite surface interne (6) dudit corps annulaire (1), qui est située en aval des moyens supports (8) du corps annulaire (1), prend la forme d'une surface de confusion (30) coopérant avec les projections (20) qui interagissent avec les surfaces supports en aval (10) desdits moyens supports (8).

6. Prothèse de valve cardiaque selon l'une quelconque des revendications 1 à 5, **caractérisée en ce que** ledit moyen support (8) est disposé le long de la périphérie entière dudit corps annulaire (1), au moins une partie (18) de la surface en aval (17) de chaque feuillet (13) étant régulièrement concave afin de permettre le passage du flux sanguin direct (4) entre les feuillets (13) dans leur position ouverte.

7. Prothèse de valve cardiaque selon l'une quelconque des revendications 1 à 5, **caractérisée en ce que** ledit moyen de support (8) est formé par deux protubérances (27) situées sur des portions diamétralement opposées de ladite surface d'extrémité en amont (2) dudit corps annulaire (1), les surfaces latérales (11) des deux protubérances (27) faisant face l'une à l'autre et les projections desdites surfaces latérales (11) sur un plan perpendiculaire à l'axe central (12) dudit corps annulaire (1) étant des arcs d'un cercle dont le centre est situé sur ledit axe central (12).

8. Prothèse de valve cardiaque selon la revendication 7, **caractérisée en ce que**, au niveau des extrémités de la surface support en amont (10) de chaque protubérance (27), des butées (28) sont prévues pour coopérer avec lesdites projections (21) interagissant avec lesdites surfaces supports en amont (10), la longueur de chaque surface en amont (10) entre lesdites butées (28) s'échelonnant de 0,05 à 0,25 fois la longueur de la surface interne (6) dudit corps annulaire (1).

9. Prothèse de valve cardiaque selon la revendication 1, **caractérisée en ce que**, dans au moins un desdits feuillets (13), un canal (31) est prévu, la surface du canal (31) traversant la surface de contact (14) dudit feuillet (13) afin de permettre un reflux sanguin restreint (5) lorsque les feuillets (13) sont dans la position fermée.

10. Prothèse de valve cardiaque selon la revendication 9, **caractérisée en ce que**, au moins un canal (31) est prévu au niveau de la surface de contact (14) de chaque feuillet (13), le canal (31) étant délimité d'un côté par la surface interne (6) dudit corps annulaire (1), et **en ce que** la surface globale dudit au moins un canal (31) et des dégagements (33) entre la surface latérale externe (15) des feuillets (13) et la surface interne (6) dudit corps annulaire (1) s'échelonne de 0,2 à 8% de la surface d'orifice dudit corps annulaire (1).
